# EUROPEAN PATENT APPLICATION

(11) **EP 1 273 302 A2**
(43) Date of publication of application: **08.01.2003**
(21) Application number: 02380116.0
(22) Date of filing: 06.06.2002
(51) Int. Cl.: A61K 35/78

(54) **A composition for weight loss and obesity control**

(30) Priority: 05.07.2001 ES 200101561
(71) Applicant: Madaus, S.A., 08038 Barcelona (ES)
(72) Inventor: Redondo Marquez, Luis, 08038 Barcelona (ES)
(74) Representative: SUGRANES - VERDONCES - FERREGÜELA

(57) **Abstract**

The composition includes a mixture of Glucomannan and Plantago Ovata as its active ingredients. The Plantago Ovata is included in the form of seed husks (Ispaghula husk). The optimum proportion between Glucomannan and Plantago Ovata is approximately 1 to 6 by weight.

## Description

### Technical field of the invention

The present invention refers to a composition for weight loss and obesity control, of the type that includes Glucomannan as one of its active substances.

### State of the art

Obesity is a public health problem as it affects a large percentage of the population and, at the same time, determines an increase in the morbidity and mortality of those individuals who suffer from it. The prevalence of obesity seems to increase alarmingly in the majority of developed countries. It is for this reason that there is great interest in delimiting strategies to prevent or control this pathology that is associated with metabolic, physical, psychological and social complications.

Obesity is frequently associated with other metabolic disturbances, involving, in this case, a disproportionate increase in the risk of suffering from cardiovascular diseases. Amongst these metabolic complications are insulinic resistance or diabetes, situations classified as high risk in the handbooks concerning the prevention and treatment of this disease.

At present, diet can be considered as the basis for all treatments of both obesity control and associated diabetes. Amongst the dietetic factors included in the treatment of these situations are a moderate reduction in caloric provision especially as fats, a reduction in caloric density of the food and the intake of an amount of fibre which covers the recommended needs.

Different arguments support the necessity to recommend fibre in the diet of the obese and diabetic patient. Obesity and diabetes are very rare in populations in developing countries which ingest a large quantity of fibre. However, developed countries tend to ingest less and less complex carbohydrates and fibre, whilst the prevalence of obesity and type II diabetes increases. On the other hand, the vegetarian population has less prevalence of obesity within the same country, suggesting that fibre intake could play a role both in the prevention and treatment of obesity and diabetes.

Different authors have suggested the possible mechanisms that explain the relation between obesity and fibre intake. On the one hand, fibre rich diets have little energetic density, producing by volume effect, and, due to greater mastication time, an increase in satiety. Likewise, fibre is associated with a delay in gastric emptying, also producing a delay in the sensation of hunger. Furthermore, fibre produces a reduction in the digestibility of proteins and carbohydrates, thereby increasing faecal energy losses. Finally, fibre intake produces a reduction in postprandial insulin secretion, thus increasing satiety.

The American Diabetes Association and other scientific organisations now recommend that diabetics consume diets containing between 30 and 40 g of fibre daily, even recognising that the amount of fibre necessary to improve the glycaemic profile is between 2 to 3 times greater than the amount of fibre consumed by the population of many developed countries.

Diet in diabetes mellitus pursues, amongst other things, glycaemic control, both on an empty stomach and in postprandial situations, as it has been associated with a considerable reduction in mortality due to heart disease and other causes as well as long term microvasculatory complications. Among the dietetic strategies that achieve more acceptable postprandial glycaemia levels, one can include the use of fibre rich diets with low glycaemic indexes, which possibly act by slowing down carbohydrate absorption. Population studies have demonstrated that this type of diet plays a protective role in type II diabetes and cardiovascular disease prevention.

Different authors have demonstrated that the addition of different specific types of non-digestible fibre improves glucose tolerance in healthy volunteers and in obese and diabetic patients, whether or not they are treated with insulin.

Until now, the most widely used fibres in our country for glycaemic control or hypolipemiant agents have been Guar fibre and Glucomannan, both highly viscous.

As examples of Glucomannan use for said purposes, the following patents can be mentioned: WO 90/07879, WO 90/07880, WO 91/00694 and WO 91/00695 of DICOFARM, S.p.A, the European patent 0 479 126 of UNI COLLOID KABUSHIKI KAISHA and the USA patent 4,844,913 of KABUSHIKI KAISHA MARUZEN SHOKUHIN and KABUSHIKI KAISHA SOKENSHA, in which Glucomannan is used in different forms.

Nevertheless, some of the fibres studied, such as Glucomannan, have, due to their high fermentability, difficulties in the patient's therapeutic performance, as they very frequently cause flatulence, creating an uncomfortable situation for the person consuming it.

### Explanation of the invention.

To avoid said inconveniences and for the purpose of reducing this secondary effect, a preparation based on two fibres (Plantago Ovata and Glucomannan) which have separately demonstrated beneficial effects in glycaemia figure controls, was designed so that joint consumption might permit reducing the dose of one of them, Glucomannan, in this way reducing its rapid, high fermentability. Thus, the present invention proposes the combination of two natural active ingredients, Glucomannan and Plantago Ovata, with proven individual effectiveness both in the treatment of disregulations of intestinal disorders, and in lipid and carbohydrate absorption. If the satiating effect of these fibres and consequent reduction of appetite sensation is added to this, this combination constitutes a new therapy with excellent possibilities for success in the coadjuvant treatment of obesity and type II diabetes mellitus.

The two active ingredients contained in the composition which is the object of the invention, are widely known and are currently described in numerous International Pharmacopoeias.

Glucomannan is a polysaccharide constituted from glucose and mannose units, which form part of the soluble fibres group, extracted from the tubers of a species from the arum family. They have a high hydration volume compared with other substances such as carboxymethylcellulose, wheat bran and pectin. This property remains unchanged in the presence of possible pH alterations. Studies carried out on the physiochemical properties of different fibres in relation to gastrointestinal function have shown that Glucomannan presents a high absorption capacity and high viscosity, in addition to low ionic exchange capacity, which converts it into the ideal fibre to regulate glucose and cholesterol levels. This capacity to absorb large quantities of water produces a sensation of satiety, as the water molecules that are incorporated in the polysaccharide chain cause them to swell.

Through the same mechanism, Glucomannan can absorb certain exogenous molecules, such as lipids, sterols and even sugars in the gastrointestinal tract, eliminating them from the organism through an increase in excretion. This property of Glucomannan clarifies its use in body weight control.

Glucomannan remains unchanged in the human intestine, it is not absorbed or hydrolysed and undergoes partial degradation in the colon.

The most recent studies demonstrate that Glucomannan controls the appetite, prolonging postprandial satiety, reducing calorie absorption and lipogenesis and avoiding lipolysis inhibition.

Nevertheless, treatment with Glucomannan or Guar is accompanied by poor performance over time, very probably due to the fact they are rapidly fermented in the intestine, producing large quantities of volatile fatty acids. On the other hand, Plantago Ovata husks together with small quantities of Glucomannan seem to ferment more slowly.

Essentially, the composition for weight loss and obesity control, which is the object of the present invention, is characterized in that it comprises a mixture of Glucomannan and Plantago Ovata as active ingredients.

According to another characteristic of the composition of the invention, Plantago Ovata is included in the form of seed husks (Ispaghula husk).

In accordance with another characteristic of the composition of the invention, the optimum proportion between Glucomannan and Plantago Ovata is approximately 1 to 6 by weight.

Plantago Ovata (PO) is a plant originating from Africa and Asia, belonging to the zaragotana family. Specifically, both the PO seeds and husks are used in pharmaceutical products. The husks receive the name Ispaghula husk or Psyllium husk.

The PO seeds contain soluble and insoluble fibre in a 20/80 ratio, i.e. there is more insoluble than soluble fibre. On the contrary, in the husks, the ratio is 70/30, i.e. higher soluble than insoluble quantity.

PO has been attributed with different pharmacological properties, of which the best known is its capacity to normalise the intestinal tract. This property is not only due to its capacity to trap water and consequently increase its volume, but also to other mechanisms that, associated with this, manage to normalise the transit time of the faeces through the colon and their consistency.

Another property of both the PO seeds and husks is their capacity to be fermented by colon bacteria. The studies carried out show that the seeds produce much greater non-digestible remains than the husks, i.e. the most fermentable part corresponds to the most soluble parts of the PO. In consequence, PO offers a part which is fermentable in the colon, with the resulting benefits derived from this fermentation (short chain fatty acids production).

It has also been described that OP inhibits the bacterial β-glucuronidase enzyme. Different studies have demonstrated that a rise in this enzyme's activity causes an increase in the incidence of colorectal tumours and that the inhibitors of this enzyme may reduce this risk. In a study done on rats, it was shown that PO significantly inhibited the activity of β-glucuronidase in the intestine.

Another of the actions described for PO is its capacity to increase biliary acid elimination through faeces. Experimental studies have clearly demonstrated that PO seeds, and, above all, their husks, increase biliary acid elimination through faeces.

As regards lipidic and glycaemic metabolism, PO also has a noteworthy action, in the sense that it reduces seric cholesterol levels, as numerous clinical and experimental studies have demonstrated. The mechanism of the action could be, amongst others, an increase in biliary acid elimination and the consequent stimulation of its production from cholesterol. More recent studies show a reduction of the total cholesterol and LDL fraction and an increase in HDL, achieving with this an improved lipidic profile.

Furthermore, PO improves the postprandial glucose curve in diabetic patients, especially those who are not insulin dependent (type II). This effect has been shown in numerous experimental studies, where the husks have developed the greatest activity. Recently, various studies have been published where PO husks are demonstrated to reduce basal and postprandial glycaemia levels.

For this purpose, a study was carried out to evaluate in vitro production of short chain fatty acids (SCFA) in healthy volunteers, after the incubation of fresh faeces (< 45 minutes after defecation), with different types of fibre: PO seeds, PO husks plus Glucomannan, and Glucomannan. The results obtained have demonstrated that Glucomannan is the fibre which produces greatest quantities of SCFA (evaluated in values of the Area Under the Curve in 24 hours), showing that after just 6 hours there are considerable differences between the fermentation levels of Glucomannan and the PO and Glucomannan combination (Figure 1).

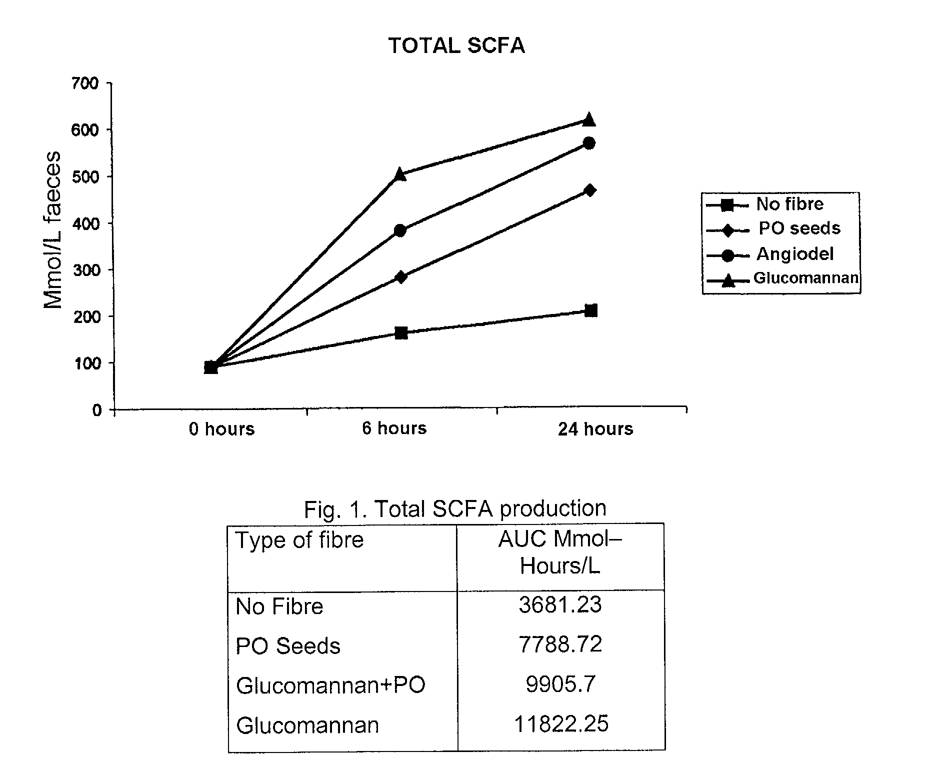

Furthermore, a study on healthy volunteers has been carried out to evaluate the effects of administering the Plantago Ovata + Glucomannan mixture, according to the invention, on the area under the glycaemia curve post administration of 75 grams of glucose, in comparison with the administration of just Glucomannan.

This study had a crossed design so that the volunteers randomly received the two types of treatment. Each phase of the treatment consisted in either 1 g of Glucomannan or 1 g of Glucomannan together with 6 g of Plantago Ovata.

The results have demonstrated that both the area under the curve (AUC) and the maximum peak of the glycaemia post loading glucose were significantly higher in the group who received just Glucomannan, in comparison with the group who received the Glucomannan and Plantago Ovata mixture according to the invention. (AUC p =0.03; maximum peak p = 0.01).

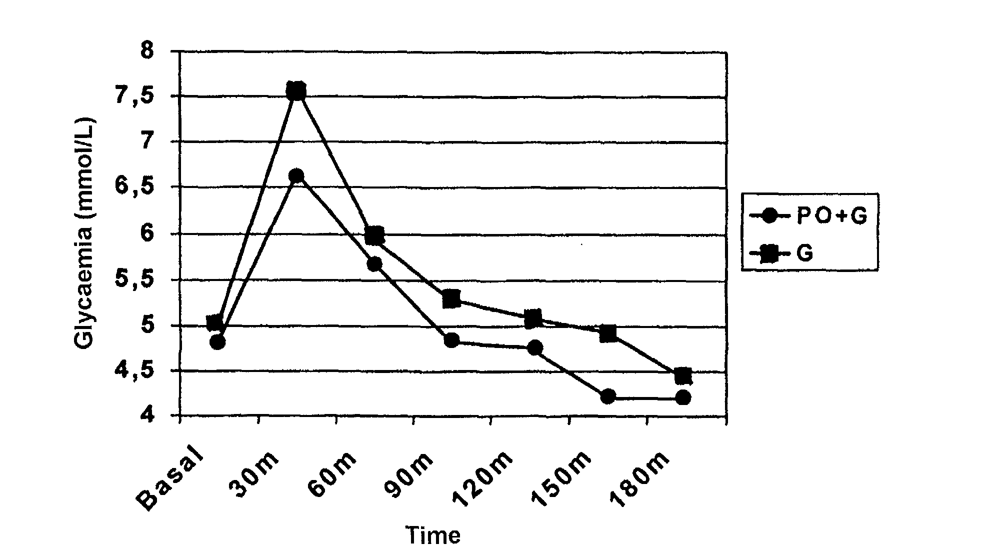

Figure 2: Glycaemic response post loading glucose after taking each of the treatments (G= Glucomannan; P+G= Plantago Ovata + Glucomannan)

Finally, a practical example of the pharmaceutical composition, which is the object of this invention, is included.

### Example.-

Effervescent composition in single dose packets with 5.5 g of effervescent powder.

### Active ingredients:

| | |
|---|---|
| Plantago ovata seed husks | 3.000 g |
| Glucomannan | 0.500 g |

### Excipients:

| | |
|---|---|
| Sodium bicarbonate | 0.884 g |
| Granulated tartaric acid | 0.930 g |
| Ground sodium saccharine | 0.030 g |
| Pal Super orange colouring | 0.006 g |
| Orange nuclearoma 32 N-3 | 0.150 g |

## Claims

1. Composition for weight loss and obesity control, **characterized in that** it includes a mixture of Glucomannan and Plantago Ovata as active ingredients.

2. Composition according to claim 1, **characterized in that** Plantago Ovata is included in the form of seed husks (Ispaghula husk).

3. Composition according to any one of the previous claims, **characterized in that** the optimum proportion between Glucomannan and Plantago Ovata is approximately 1 to 6 by weight.
